(19) Europäisches Patentamt
European Patent Office
Office européen des brevets

(11) Numéro de publication: **0 424 829 A1**

(12) **DEMANDE DE BREVET EUROPEEN**

(21) Numéro de dépôt: **90120132.7**

(51) Int. Cl.[5]: **C07C 255/58**, C07C 255/54, C07C 323/62, G02F 1/35

(22) Date de dépôt: **19.10.90**

(30) Priorité: **26.10.89 FR 8914062**

(43) Date de publication de la demande: **02.05.91 Bulletin 91/18**

(84) Etats contractants désignés: **DE FR GB IT NL**

(71) Demandeur: **ALCATEL N.V.**
**Strawinskylaan 341 (World Trade Center)**
**NL-1077 XX Amsterdam(NL)**

(72) Inventeur: **Fauvarque, Jean-Francois**
**6, rue Joseph Bara**
**F-75006 Paris(FR)**
Inventeur: **Jutand, Anny**
**25, rue Mouton Duvernet**
**F-75014 Paris(FR)**
Inventeur: **Amatore, Christian**
**39, rue Dunois**
**F-75013 Paris(FR)**
Inventeur: **Negri, Serge**
**43, rue de Liers**
**F-91240 Saint Michel sur Orge(FR)**

(74) Mandataire: **Weinmiller, Jürgen et al**
**Lennéstrasse 9 Postfach 24**
**W-8133 Feldafing(DE)**

(54) **Matériau organique pour l'optique non linéaire et les dispositifs électro-optiques.**

(57) Matériau organique pour l'optique non linéaire et les dispositifs électro-optiques, dont la molécule comprend dans sa structure un groupe donneur d'électrons D et un groupe attracteur d'électrons A reliés par l'intermédiaire d'un système transmetteur d'électrons, caractérisé par le fait qu'il répond à la formule

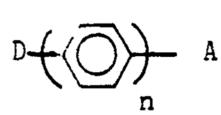

avec n égal à 3 ou 4 et A = CN.

EP 0 424 829 A1

## MATÉRIAU ORGANIQUE POUR L'OPTIQUE NON LINÉAIRE ET LES DISPOSITIFS ÉLECTRO-OPTIQUES.

La présente invention concerne un matériau organique pour l'optique non linéaire et les dispositifs électro-optiques..

Les matériaux présentant des propriétés non linéaires en optique peuvent être utilisés pour de nombreuses applications : doubleurs de fréquence, bistables optiques, commutateurs, modulateurs, coupleurs directifs, amplificateurs paramétriques etc...

On connaît un petit nombre de matériaux inorganiques non isotropes, et en particulier non centrosymétriques, qui possèdent un coefficient quadratique non nul et des propriétés non linéaires en optique ; il s'agit notamment du potassium-dihydrogène-phosphate (KDP), du nioabate et du tantalate de lithium ($LiNbO_3$, $LiTaO_3$)...

La mise en oeuvre de ces matériaux inorganiques est souvent difficile ; c'est pourquoi on s'est intéressé à des matériaux organiques à propriétés non linéaires, en particulier quand il s'agit de matériaux polymériques, thermoplastiques ou filmogènes.

La non linéarité optique est généralement obtenue en incorporant, par mélange ou greffage chimique dans le matériau organique des molécules ou groupements moléculaires polaries fortement hyperpolarisables.

De telles molécules sont synthétisées en associant un groupe attracteur d'électrons à un groupe donneur d'électrons par l'intermédiaire d'un système transmetteur d'effets électroniques.

On connaît par exemple les matériaux suivants :

- La paranitroaniline (PAN)

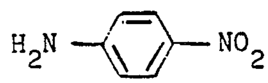

- Le N (nitro 4 phényl) N méthyl amino 2 acétonitrile (NPAN)

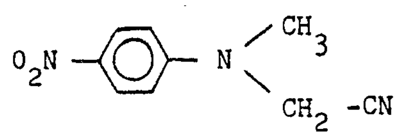

- La dinitro 2-4 phényl L alanine (MAP)

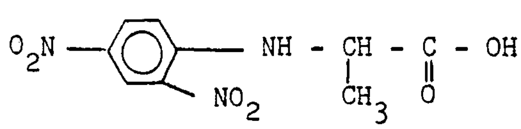

- Le méthyl 3 nitro 4 pyridine N-oxyde (POM)

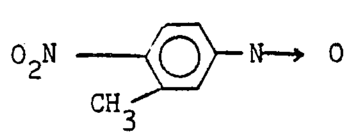

Ces molécules sont caractérisée par leur coefficient d'hyperpolarisabilité défini par le développement en série du moment dipolaire

$$\vec{\mu}$$

en fonction du champ électrique E.

$$\vec{\mu} = \vec{\mu}_0 + \alpha.\vec{E} + \beta|\vec{E}|^2 + \ldots$$

Plus le coefficient $\beta$ est élevé, plus les molécules présentent de l'intérêt pour l'optique non linéaire. Le coefficient $\beta$ peut être obtenu par la méthode EFISH (Electric Field Induced Second Harmonic generation). Ce coefficient $\beta$ peut être augmenté en allongeant le système de doubles liaisons conjuguées entre le donneur et l'accepteur. Cette augmentation se traduit également par un déplacement vers le rouge des bandes d'absorption optiques, qui peut devenir préjudiciable à l'utilisation des matériaux dans le visible ou le proche infrarouge.

La présente invention a pour but de pallier ces inconvénients.

La présente invention a pour objet un matériau organique pour l'optique non linéaire et les dispositifs électro-optiques, dont la molécule comprend dans sa structure un groupe donneur d'électrons D et un groupe attracteur d'électrons constitué par le radical CN, reliés par l'intermédiaire d'un système transmetteur d'électrons, caractérisé par le fait qu'il répond à la formule

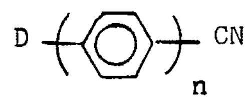

avec n égal à 3 ou à 4.

Le matériau selon l'invention comporte des molécules faiblement conjuguées à l'état fondamental, peu absorbantes dans le visible et dans le proche infrarouge, et qui possèdent cependant des coefficients $\beta$ très élevés dans le proche infrarouge, qualité nécessaire à l'utilisation de ces matériaux dans des dispositifs électro-optiques.

Selon un mode de réalisation préférentiel le groupe donneur d'électrons D est choisi parmi $(CH_3)_2N$, $CH_3O$, $CH_3S$.

La présente invention a également pour objet un procédé de préparation des matériaux précédents, caractérisé par le fait que l'on effectue la synthèse :

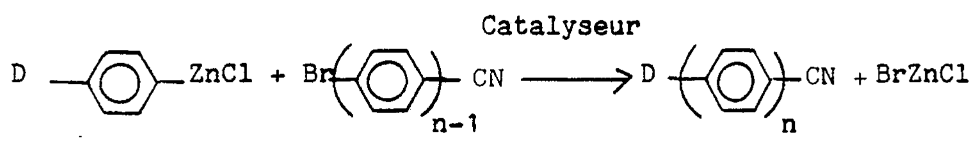


ledit catalyseur étant un complexe à bas degré d'oxydation d'un métal de transition, par exemple du nickel, du palladium, du platine, ou un ion métallique isoélectronique.

De préférence, on effectue la synthèse de

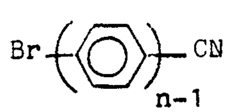 suivant la réaction :

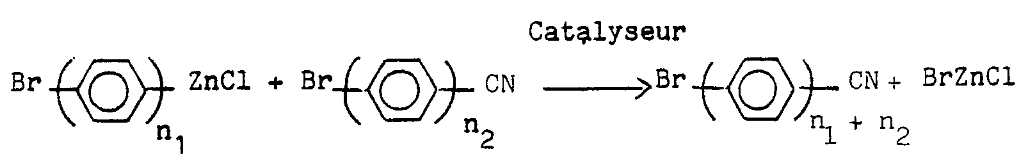


avec $n_1 + n_2 = n-1$

Selon un mode préférentiel, on obtient

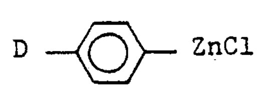

de la manière suivante :
on part de magnésium en suspension dans du tétrahydrofuranne distillé, on ajoute à température ordinaire une solution de D

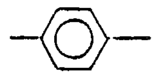

Br dans du tétrahydrofuranne anhydre, on ajoute à l'organomagnésium ainsi formé une solution de $ZnCl_2$ deshydraté dans du tétrahydrofuranne selon la réaction :

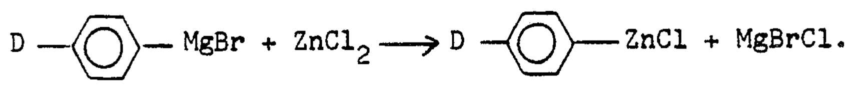

D'autres caractéristiques et avantages de la présente invention apparaîtront au cours de la description suivante de modes de réalisation donnés à titre illustratif mais non limitatif.

Pour préparer un matériau selon l'invention de type

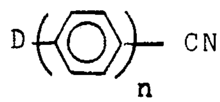

avec n = 3 ou 4,
on a besoin d'un précurseur de type

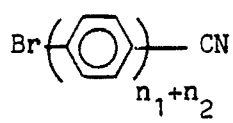

avec $n_1 + n_2 = n-1$, obtenu de la manière suivante :

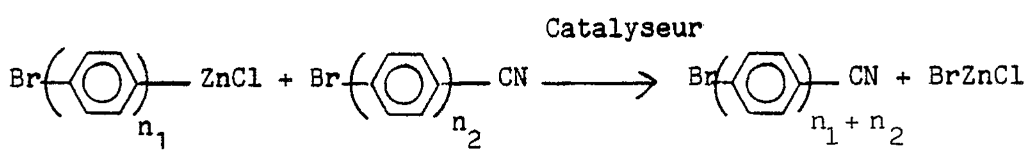


Le catalyseur est de type $Pd^0 (PPh_3)_4$, c'est-à-dire du tetrakis triphényphosphine palladium.

Trois exemples de réactions sont schématisés dans le tableau I ci-dessous.

TABLEAU I

| $n_1$ | $n_2$ | 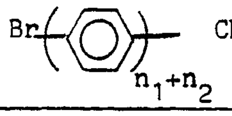 |
|---|---|---|
| 1 | 1 | 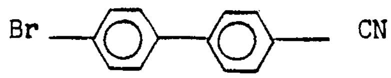 |
| 1 | 2 | 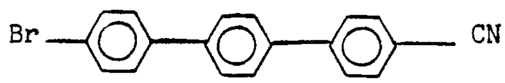 |
| 2 | 1 | // |

La première réaction a lieu en présence de 2,5% de catalyseur pendant 12 heures à 20° C, puis pendant 2 heures à 60° C.

La seconde réaction a lieu en présence de 10% de catalyseur pendant 24 heures à 20° C.

La troisième réaction a lieu en présence de 2% de catalyseur pendant 24 heures à 20° C.

Ces précurseurs sont utilisés dans les trois exemples suivants :

EXEMPLE I :

synthèse de

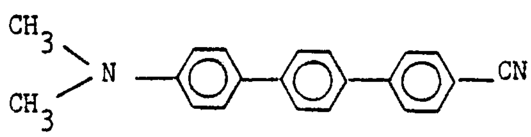

Dans une première étape on réalise

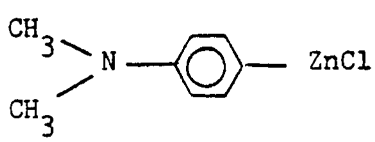

A 0,148 g de magnésium en suspension dans 1 ml de THF distillé, on ajoute une solution de 1,2 g de

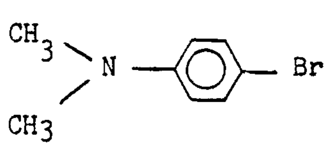

dans 4 ml de THF anhydre. La réaction est faite à température ambiante. L'organomagnésien est ensuite ajouté dans une solution de 0,831 g de $ZnCl_2$ deshydraté dans 5 ml de THF. Cette réaction conduit à la formation du zincique correspondant :

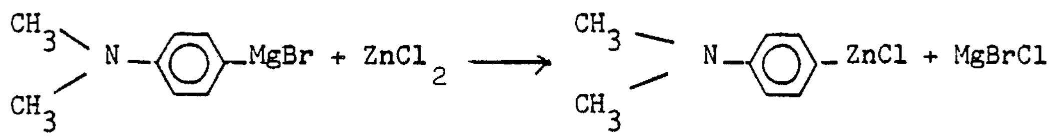

Dans une seconde étape on met en oeuvre la réaction :

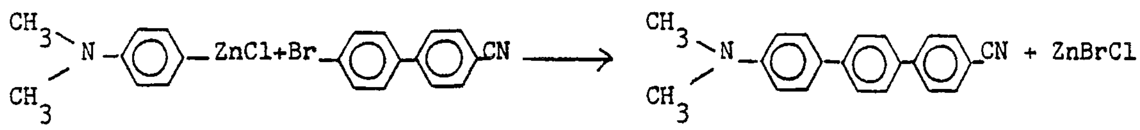

La solution d'organozincique obtenue à la première étape est ajoutée à une solution de 5 ml de THF contenant 1,29 g de

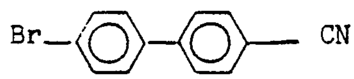

et 0,578 g de catalyseur Pd $(PPh_3)_4$.

Après une nuit à température ambiante, le produit de la réaction précipite. Il est lavé à l'eau puis rincé à l'éthanol. Après recristallisation dans le chlorure de méthylène on récupère 0,81 g de :

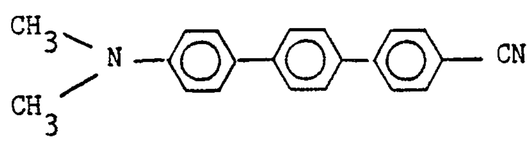

avec un rendement de 54%.

Ce matériau présente un coefficient d'hyperpolarisabilité $\beta$ tel que $\beta_0$ = 48 x $10^{-30}$ esu
$\beta$1.34 nm = 70 x $10^{-30}$ esu

<u>EXEMPLE II :</u>

synthèse de

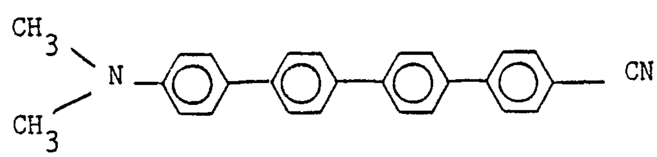

Dans une première étape on réalise

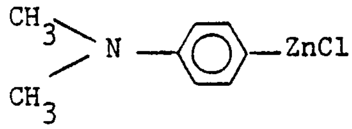

comme dans l'exemple 1.

Dans une seconde étape on met en oeuvre la réaction

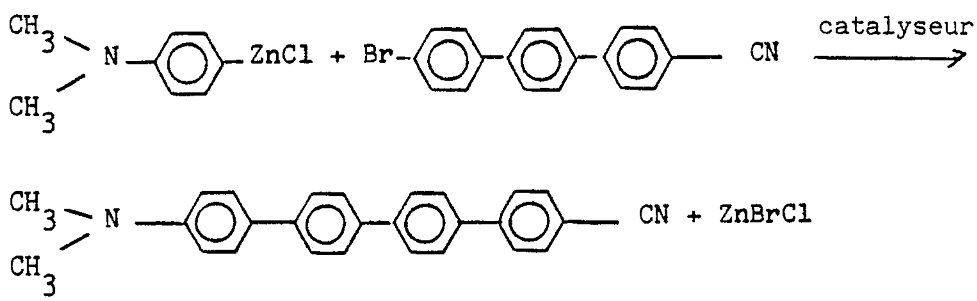


La solution d'organozincique obtenue dans la première étape est ajoutée à une solution de 5 ml de THF contenant 1,67 g de

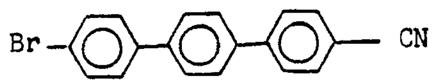

et 0,578 g de Pd $(PPh_3)_4$. Après une nuit à température ambiante et deux heures au reflux du THF, la solution est filtrée, lavée à l'eau et à l'éthanol. On récupère 1,026 g de

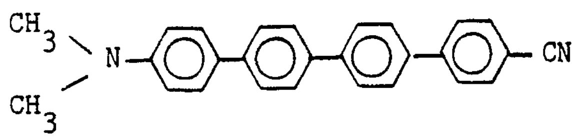

avec un rendement de 55%. Il n'a pas été possible de recristalliser le produit.

Ce matériau présente un coefficient d'hyperpolarisabilité $\beta_0 = 50 \pm 10 \times 10^{-30}$ esu.

EXEMPLE III :

Synthèse de

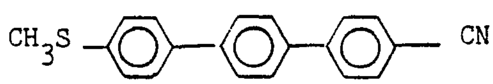

Dans une première étape on réalise

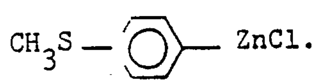

A 0,065 g de magnésium en suspension dans 1 ml de THF distillé, on ajoute une solution de 0,528 g de

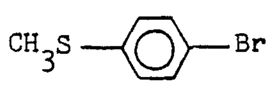

dans 2 ml de THF anhydre. La réaction est faite à température ambiante. L'organomagnésien est ensuite ajouté dans une solution de 0,367 g de $ZnCl_2$ deshydraté dans 5 ml de THF. Cette réaction conduit à la formation de zincique correspondant :

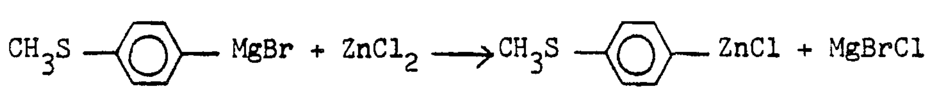

Dans une seconde étape on met en oeuvre la réaction :

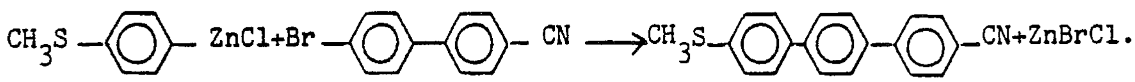

La solution d'organozincique préparée dans la première étape est ajoutée à une solution de 5 ml de THF contenant 0,645 g de

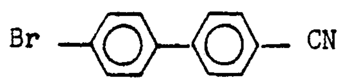

et 0,289 g de $Pd^0$ $(PPH_3)_4$. Après deux heures au reflux de THF, le milieu est versé dans 150 cc d'eau. Le solide est filtré, lavé à l'eau, à l'éther de pétrole et à l'éthanol.

On récupère 0,51 g de

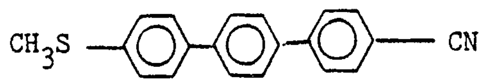

avec un rendement de 69%.

Parmi les complexes de métaux de transition utilisés comme catalyseurs, ceux qui associent des phosphines substituées et du palladium zérovalent sont particulièrement efficaces. Ceux du nickel zérovalent peuvent être parfois encore plus efficaces.

Les terphényles ou quaterphényles substitués selon l'invention, incorporés par mélange ou par greffage approprié dans des matériaux organiques, permettent la réalisation de commutateurs ou d'électro-modulateurs plus efficaces que ceux réalisés actuellement avec du niobate de lithium ou des produits analogues.

## Revendications

1/ Matériau organique pour l'optique non linéaire et les dispositifs électro-optiques, dont la molécule comprend dans sa structure un groupe donneur d'électrons D et un groupe attracteur d'électrons constitué par le radical CN, reliés par l'intermédiaire d'un système transmetteru d'électrons, caractérisé par le fait qu'il répond à la formule

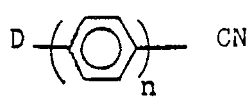

avec n égal à 3 ou 4.

2/ Matériau organique pour l'optique non linéaire selon la revendication 1 , caractérisé par le fait que ledit groupe donneur d'électrons D est choisi parmi $(CH_3)_2N$, $CH_3O$ et $CH_3S$.

3/ Procédé de fabrication d'un matériau selon la revendication 1, caractérisé par le fait que l'on effectue la synthèse

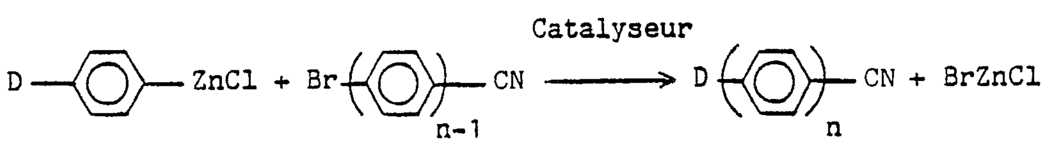

ledit catalyseur étant un complexe d'un métal de transition à bas degré d'oxydation.

4/ Procédé de fabrication selon la revendication 4, caractérisé par le fait que l'on effectue la synthèse de

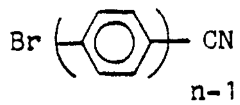

suivant la réaction :

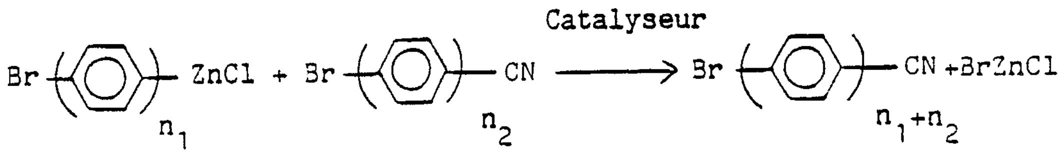


avec $n_1 + n_2 = n-1$ ledit catalyseur étant un complexe d'un métal de transition à bas degré d'oxydation.

5/ Procédé de fabrication selon la revendication 3, caractérisé par le fait que pour obtenir

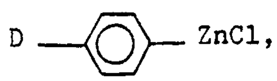

on part de magnésium en suspensiond ans du tétrahydrofuranne distillé, on ajoute à température ordinaire une solution de

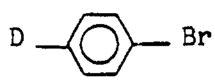

dans du tétrahydrofuranne anhydre, on ajoute à l'organomagnésium ainsi formé une solution de $ZnCl_2$ deshydraté dans du tétrahydrofuranne selon la réaction :

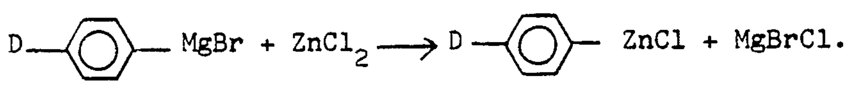

6/ Procédé de fabrication selon l'une des revendications 3 à 5, caractérisé par le fait que ledit catalyseur est $Pd^0 (PPh_3)_4$.

Office européen des brevets

# RAPPORT DE RECHERCHE EUROPEENNE

Numéro de la demande

EP 90 12 0132

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 284 229 (CANON)<br>* Revendications; exemple 4 * | 1,2 | C 07 C 255/58<br>C 07 C 255/54<br>C 07 C 323/62<br>G 02 F 1/35 |
| A | DE-A-3 704 878 (MERCK PATENT)<br>* Revendications; exemple 1 * | 1,2 | |
| X | CHEMICAL ABSTRACTS, vol. 109, CHEMICAL SUBSTANCE INDEX, R-Z, partie 6, juillet-décembre 1988<br>* Page 9390CS. [1,1':4',1"-terphenyl]-4-carbonitrile, 4"-pentyl, second order nonlinear polarizability and mol. orientation of, on water substrate * & CHEMICAL ABSTRACTS, vol. 109, no. 13, 26 septembre 1988, pages 561-562, résumé no. 109791d, Columbus, Ohio, US; G. BERKOVIC et al.: "The effect of conjugation lenght and electron donor groups on the second order nonlinear polarizability of cyano substituted aromatic molecules", & MOL. CRYST. LIQ. CRYST. 1987, 150B, 607-16 | 1,2 | |
| A | JOURNAL OF THE CHEMICAL SOCIETY, PERKIN TRANSACTIONS II, A JOURNAL PHYSICAL ORGANIC CHEMISTRY, no. 9, septembre 1987, pages 1351-1355, Royal Society of Chemistry; J.O. MORLEY et al.: "Non-linear optical properties of organic molecules. Part 2. Effect of conjugation lenght and molecular volume on the calculated hyperpolarisabilities of polyphenyls and polyenes"<br>* Pages 1352,1353; abrégé * | 1,2 | DOMAINES TECHNIQUES RECHERCHES (Int. Cl.5)<br>C 07 C 255/00<br>C 07 C 323/00<br>G 02 F 1/00 |
| A | CHEMICAL ABSTRACTS, vol. 98, no. 19, 9 mai 1983, page 459, résumé no. 160081d, Columbus, Ohio, US; B.M. KRASOVITSKII et al.: "Study of 2,5-diaryl-1,3,4-oxadiazoles. 2. Electronic structure and spectral-luminescent properties of 4-nitro-4'-dimethylamino-substituted 2,5-diphenyl-1,3,4-oxadiazole, trans-stilbene, and p-terphenyl",<br>& KHIM. GETEROTSIKL. SOEDIN. 1983, (1), 33-6 | 1,2 | |

-/-

Le présent rapport de recherche a été établi pour toutes les revendications

| Lieu de la recherche | Date d'achèvement de la recherche | Examinateur |
|---|---|---|
| La Haye | 28 janvier 91 | WRIGHT M.W. |

CATEGORIE DES DOCUMENTS CITES

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention

E: document de brevet antérieur, mais publié à la date de dépôt ou après cette date
D: cité dans la demande
L: cité pour d'autres raisons

&: membre de la même famille, document correspondant

Office européen des brevets

# RAPPORT DE RECHERCHE EUROPEENNE

Numéro de la demande

**EP 90 12 0132**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | JOURNAL OF ORGANIC CHEMISTRY, vol. 42, no. 10, 13 mai 1977, pages 1821-1823; E.-I. NEGISHI et al.: "Selective carbon-carbon bond formation via transition metal catalysis. 3. A highly selective synthesis of unsymmetrical biaryls and diarylmethanes by the nickel- or palladium-catalyzed reaction of aryl- and benzylzinc derivatives with aryl halides" * Tableau I * | 3-6 | |
| | ----- | | |
| | | | DOMAINES TECHNIQUES RECHERCHES (Int. Cl.5) |

Le présent rapport de recherche a été établi pour toutes les revendications

| Lieu de la recherche | Date d'achèvement de la recherche | Examinateur |
|---|---|---|
| La Haye | 28 janvier 91 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

&: membre de la même famille, document correspondant